# EUROPEAN PATENT SPECIFICATION

(11) **EP 3 735 231 B1**
(45) Date of publication and mention of the grant of the patent: **25.11.2026**
(21) Application number: 19700428.6
(22) Date of filing: 02.01.2019
(51) Int. Cl.: A61K 9/48, A61K 31/352, A61K 47/10

(54) **ORAL PHARMACEUTICAL FORMULATION COMPRISING CANNABINOIDS AND POLOXAMER**
ORALE PHARMAZEUTISCHE FORMULIERUNG ENTHALTEND CANNABINOIDE UND POLOXAMER
FORMULATION PHARMACEUTIQUE ORALE CONTENANT DES CANNABINOIDES ET DU POLOXAMER

(30) Priority: 03.01.2018 GB 201800074
(43) Date of publication of application: 11.11.2020
(73) Proprietor: Jazz Pharmaceuticals Research UK Limited, Sittingbourne Kent ME9 8AG (GB)
(72) Inventor: WILKHU, Jitinder, Sittingbourne, Kent ME9 8AG (GB); BENDER, Johan, 6572 AB Berg en Dal (NL)
(74) Representative: HGF
(86) International application number: PCT/GB2019/050009
(87) International publication number: WO 2019/135077

(56) References cited:
- WO-A1-2016/022936
- WO-A1-2017/059859
- WO-A1-2017/072774
- WO-A1-2018/002665
- P. CHIU ET AL: "The influence of delta9-tetrahydrocannabinol, cannabinol and cannabidiol on tissue oxygen consumption", RES COMMUN CHEM PATHOL PHARMACOL., vol. 12, no. 2, 1 October 1975 (1975-10-01), pages 267 - 286, XP055564846

## Description

### Field of the Invention

The present invention relates to an oral pharmaceutical formulation comprising a combination of at least two cannabinoids. The cannabinoids are tetrahydrocannabinol (THC) and cannabidiol (CBD).

### Background of the Invention

Cannabinoids are lipophilic substances that are known to be poorly soluble in water (less than 1 µg/mL). As an example, CBD is soluble in ethanol (36 mg/mL) and dimethylsulfoxide DMSO (60 mg/mL).

Bioavailability of pharmaceutical substances taken perorally, first of all, depends on the extent to which the pharmaceutically active substance is absorbed from the intestinal environment across the intestinal mucosa. Lipophilic pharmaceutical substances are generally poorly absorbed from the intestinal environment, inter alia because of their poor solubility and/or dispersibility in water. Bioavailability of a pharmaceutical substance taken perorally furthermore depends on the susceptibility of the substance to the so-called first pass effect. Substances absorbed from the intestine, before being distributed throughout the body, have to pass the liver first where they may be metabolised immediately. CBD is generally assumed to be rather susceptible to first-pass liver metabolisation. Oral bioavailability of CBD is low and unpredictable (S. Zhornitsky, S. Potvin, Pharmaceuticals (2012) 5, 529-552). In addition, CBD is an unstable drug (A. J. Poortman, H. Huizer, Forensic Science International (1999) 101, 1-8).

In WO 2012/033478, Self-Emulsifying Drug Delivery Systems (SEDDS) have been used to offer improved administration of cannabinoids.

SEDDS (self-emulsifying drug delivery systems) generally consist of hard or soft capsules filled with a liquid or a gel that consists of lipophilic active pharmaceutical ingredient (API), oil (to dissolve the API) and a surfactant. Upon contact with gastric fluid, the SEDDS spontaneously emulsify due to the presence of surfactants. Many surfactants, however, are lipid based and interact with lipases in the gastro intestinal tract (GIT). This can lead to a reduced capability of the lipid based surfactants to emulsify the API as well as the oil carrier, both reducing bioavailability.

In WO 2015/184127, an alcohol-free formulation comprising a cannabinoid, a polyethylene glycol and propylene glycol is disclosed.

In WO 2012/033478, SEDDS formulations based on Type I, Type II and Type III were utilised.

In WO 2018/002665 A1 (PCT/GB2017/051943) a Type IV or Type IV-like formulation comprising a cannabinoid is disclosed.

WO 2017/072774 A1 discloses a pharmaceutical composition for transmucosal administration of an active lipophilic compound through the oral mucosa comprising a lipophilic active compound, a polymeric matrix formed by two or more water-soluble polymers and a rapid dissolution agent.

WO 2017/059859 A1 discloses a medical chewing gum comprising gum base polymers and one or more cannabinoids as an active pharmaceutical ingredient, the gum base polymers comprising polyvinyl acetate and vinyl laurate-vinyl acetate copolymer in an amount of more than 90% by weight of the gum base polymers, wherein the gum base polymers include 20 - 95% by weight of polyvinyl acetate and 5 - 80% by weight of vinyl laurate-vinyl acetate copolymer.

P. Chiu, et al., "The influence of delta9-tetrahydrocannabinol, cannabinol and cannabidiol on tissue oxygen consumption", Res. Commun. Chem. Pathol. Pharmacol., 1975, 12(2), 267-286 discloses *in vitro* studies of the influence of various cannabinoid vehicles containing Tween 80, ethanol, Pluronic F68, and albumin on the oxygen consumption of tissue preparations.

WO 2016/022936 A1 discloses an oral gastrointestinal dosage form of cannabinoids and/or standardized marijuana extracts in a self-emulsifying system operable to avoid hepatic first pass metabolism via targeted chylomicron/lipoprotein delivery for promoting lymphatic transport.

Other documents relevant to the background of the present invention are CN103110582; CN101040855; US2012/183606; Thumma S Et Al, European Journal of Pharmaceutics and Biopharmaceutics. vol 70, no. 2, 1 October 2008, pp 605-614; and Edward Maa Et Al, Epilepsia, vol. 55, no. 6, 1 June 2014, pp 783-786.

The Lipid Formulation Classification System (LFCS) was introduced to help identify the characteristics of lipid systems (C.W. Pouton, Eur. J. Pharm. Sci., 11 (Suppl. 2) (2000), pp. S93-S98). As classified in the LFCS, Type I formulations are oils which require digestion, Type II formulations are water-insoluble self-emulsifying drug delivery systems (SEDDS), Type III systems are SEDDS or self-micro emulsifying drug delivery systems (SMEDDS) or self-nano emulsifying drug delivery systems (SNEDDS) which contain some water-soluble surfactants and/or co-solvents (Type IIIA) or a greater proportion of water soluble components (Type IIIB). Category Type IV represents a recent trend towards formulations which contain predominantly hydrophilic excipient surfactants and co-solvents. Below is a tabular Lipid Formulation Classification System overview taken from US 2015/111939:

| | Content of formulation (wt.-%) | | | | |
|---|---|---|---|---|---|
| Excipients in formulation | Type I | Type II | Type IIIA | Type IIIB | Type IV |
| Oil: triglycerides or mixed mono- and diglycerides | 100 | 40-80 | 40-80 | <20 | - |
| Water-insoluble surfactants (HLB < 12) | - | 20-60 | - | - | 0-20 |
| Water-soluble surfactants (HLB > 12) | - | - | 20-40 | 20-50 | 30-80 |
| Hydrophilic co-solvent | - | - | 0-40 | 20-50 | 0-50 |

A further description of the Lipid Formulation Classification System can also be found in FABAD J. Pharm. Sci., pages 55-64, 2013.

As can be seen in the above table, Type IIIB formulations comprise <20 wt% of oil, based on the total composition. However, it should be noted that, by definition, Type IIIB formulations contain some oil, even if it is only a very small amount.

Based upon the different molecular targets engaged by THC and CBD, the potency at which molecular targets proposed for mechanism of action are engaged and relative potency observed in experimental models of disease, the utility of a THC:CBD combination therapy is proposed.

CBD and THC possess different pharmacological profiles based upon their molecular target engagement and the potency with which they affect their targets. Specifically, in contrast to the nanomolar CB1 and CB2 receptor affinity and agonist activity exhibited by THC, CBD lacks such target engagement and instead interacts with a different range of distinct molecular targets in the micromolar concentration range (e.g. inhibition of adenosine and monoamine reuptake and TRPV1 and GPR55 receptor antagonism; for review see Ibeas-Bih, 2015).

The therapeutic relevance of these differences in molecular target profile and molecular target affinity are exemplified by the relative potencies of THC and CBD in models of disease.

For example, THC at between 1.25 and 10 mg/kg (Boggan et al., 1973) and CBD at 100 mg/kg, but not 1 or 10 mg/kg, (Jones et al., 2010) are efficacious in acute experimental models of generalized seizure.

On this basis, and given the relative potency of CBD and THC at disease-relevant targets and their resulting pharmacological effects in models of disease and in clinical use, the therapeutic combination of THC and CBD at ratios of between 1:25 and 1:100 represents a novel approach to the treatment of various diseases.

A similar separation of potency is observed in man where 20mg oral THC administration induces somnolence (Gorelick et al., 2013) and similar effects seen at approximately 1000mg/day with CBD (Devinsky 2017).

### Brief Summary of the Invention

The present invention relates to a novel cannabinoid oral pharmaceutical dosage form, based on a Type IV or Type IV-like formulation, as classified using the Lipid Formulation Classification System. The formulation comprises a combination of at least two cannabinoids. The first cannabinoid is tetrahydrocannabinol (THC); and the second cannabinoid is cannabidiol (CBD). By Type IV-like, it is meant that the formulation comprises no oil, for example no triglycerides or mixed glycerides. When a Type IV-like formulation is used, it may comprise more than the 50 wt% of solvent, based on the total composition, as specified in the LFCS table.

The oral pharmaceutical formulation comprises tetrahydrocannabinol (THC); cannabidiol (CBD); a solvent in an amount of 20 to 50 wt%, wherein the solvent is selected from the group consisting of diacetin, propylene glycol, triacetin, monoacetin, propylene glycol diacetate, triethyl citrate and mixtures thereof; and at least one poloxamer in an amount of 30 to 60 wt%, wherein the poloxamer is poloxamer 124 or poloxamer 188, or a mixture thereof; and wherein the formulation is oil-free.

This formulation enhances cannabinoid bioavailability compared to other formulations based on Type I, Type II, Type IIIA and Type IIIB, as classified by the Lipid Formulation Classification System. Accordingly, the oral pharmaceutical dosage form or formulation is oil-free.

By enhancing bioavailability, the total amount of cannabinoid and excipients required during a certain window of time in a treatment of a specific disease may be reduced.

The formulation according to the present invention exhibits excellent stability under various, in particular dry, storage conditions.

By enhancing stability, the length of time for which the formulations are fit for consumption, in particular oral administration, may be increased.

### Detailed Description of the Invention

### The Cannabinoid

The formulation according to the present invention comprises tetrahydrocannabinol (THC) and cannabidiol (CBD).

The formulation may comprise further cannabinoids selected from the group consisting of cannabichromene (CBC), cannabichromenic acid (CBCV), cannabigerol (CBG), cannabigerol propyl variant (CBGV), cannabicyclol (CBL), cannabinol (CBN), cannabinol propyl variant (CBNV) and cannabitriol (CBO). This list is not exhaustive and merely details the cannabinoids which are identified in the present application for reference. So far, over 100 different cannabinoids have been identified and these cannabinoids can be split into different groups as follows: Phytocannabinoids; Endocannabinoids; and Syntho-cannabinoids.

The formulation according to the present invention may also comprise at least one cannabinoid selected from those disclosed in Handbook of Cannabis, Roger Pertwee, Chapter 1, pages 3 to 15.

The cannabinoids used in the present invention may be synthetically produced or highly purified from their natural source.

Preferably the formulation is absent or substantially absent of other cannabinoids.

It is preferred that the formulation comprises only two cannabinoids, wherein the first cannabinoid is tetrahydrocannabinol (THC) and the second cannabinoid is cannabidiol (CBD).

It is preferred that the total amount of cannabinoids is in an amount of from about 5 to 80 wt%, based on the total composition, preferably from about 10 to 50 wt%, more preferably from about 20 to 30 wt%. The total cannabinoids may be present in an amount of about 30 wt%.

Preferably, the cannabinoids are synthetically produced or highly purified from their natural source (for example, plant derived recrystallized form, such as a plant derived recrystallized form of CBD). When a highly purified source is used, it is purified such that the cannabinoid is present at greater than 95%, more preferably greater than 98% of the total extract (w/w). Use of a synthetically produced or highly purified cannabinoid is advantageous because these contain relatively low amounts of wax. This assists in prevention of the formation of an oily formulation, increasing physical stability of the formulation and wettability in an aqueous environment.

The ratio by weight of the first cannabinoid to the second cannabinoid may be in the range of from 100:1 to 1:100, preferably 60:1 to 1:60.

It is preferred that the ratio by weight of the first cannabinoid to the second cannabinoid is in the range of from 20:1 to 1:20, more preferably 5:1 to 1:5. For example, the ratio by weight of the first cannabinoid to the second cannabinoid may be 1:1.

The unit dose of each cannabinoid in the oral pharmaceutical formulation may be individually in the range of from 0.001 to 350 mg, preferably 0.1 to 350 mg, more preferably 1 to 250 mg.

For example, it is envisaged that, when in tablet or capsule unit dose form, the amount of each cannabinoid present may be individually selected from 0.5, 1.5, 2, 2.5, 10, 25, 50, 100, 150, 200, 250, 300 or 350 mg.

The total amount of cannabinoid present in the formulation may be 20 to 30 wt%, based on the total composition. It has been found that the formulation is stable and is a solid at room temperature and pressure (defined herein as 20 °C and 1 atm) even when the content of cannabinoid is relatively high, such as 25, 30 or 35 wt%. Without wishing to be bound by theory, it is believed that at least one poloxamer is essential to the stability of the formulation, particularly for high cannabinoid content.

### The Solvent

The solvent is selected from the group consisting of diacetin, propylene glycol, triacetin, monoacetin, propylene glycol diacetate, triethyl citrate and mixtures thereof.

Diacetin is also known as glycerol diacetate.

Triacetin is also known as 1,2,3-triacetoxypropane, 1,2,3-triacetylglycerol or glycerol triacetate.

Monoacetin is also known as glycerol monoacetate or glycerol acetate.

Triethyl citrate is also known as citric acid ethyl ester.

Propylene glycol, propylene glycol diacetate and triethyl citrate are preferred solvents. Preferably, the solvent is triethyl citrate or propylene glycol. Triethyl citrate is preferably used.

The solvent is present in an amount of from 20 to 50 wt%. Preferably, the solvent is present in an amount of from about 20 to 30 wt%. The solvent may be present in an amount of about 25 wt%.

When the solvent used is propylene glycol, it is preferred that it is present in an amount of from about 20 to 30 wt%, based on the total composition.

When the solvent is triethyl citrate, it is preferred that it is present in an amount of from about 20 to 30 wt%.

When only one poloxamer is present, as will be described below, it is preferred that the solvent is present in an amount of from about 45 to 50 wt%, based on the total composition.

The solvent or mixture of solvents according to the claimed invention may be the only solvent in the formulation. For example, the formulation may be substantially water-free, substantially alcohol-free and/or substantially oil-free. By "substantially water-free", "substantially alcohol-free" and "substantially oil-free", it is meant that the formulation comprises less than 2 wt%, preferably less than 1 wt% water, alcohol and/or oil based on the total composition.

The formulation is preferably substantially free from ethanol. More preferably the formulation is substantially alcohol-free.

In some embodiments the formulation is used in a paediatric patient, i.e. a patient under 18 years of age. In paediatric patients, it may be preferred that the formulation is substantially alcohol-free.

The formulation is oil-free.

Therefore, the formulation does not comprise triglycerides, diglycerides or monoglycerides or mixtures thereof derived from glycerol and at least one fatty acid selected from the group consisting of caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid and mixtures thereof. The formulation does not comprise triglycerides, diglycerides or monoglycerides or mixtures thereof.

The formulation is free from hydrogenated vegetable oils, nut oils, anise oil, soybean oil, hydrogenated soybean oil, apricot kernel oil, corn oil, olive oil, peanut oil, almond oil, walnut oil, cashew oil, rice bran oil, poppy seed oil, cottonseed oil, canola oil, sesame oil, hydrogenated sesame oil, coconut oil, flaxseed oil, cinnamon oil, clove oil, nutmeg oil, coriander oil, lemon oil, orange oil, safflower oil, cocoa butter, palm oil, palm kernel oil, sunflower oil, rapeseed oil, castor oil, hydrogenated castor oil, polyoxyethylene castor oil derivatives, borage oil, beeswax, lanolin, petroleum jelly, mineral oil and light mineral oil.

The formulation is free from triglycerides, diglycerides or monoglycerides or mixtures thereof derived from glycerol and caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid and mixtures thereof, hydrogenated vegetable oils, nut oils, anise oil, soybean oil, hydrogenated soybean oil, apricot kernel oil, corn oil, olive oil, peanut oil, almond oil, walnut oil, cashew oil, rice bran oil, poppy seed oil, cottonseed oil, canola oil, sesame oil, hydrogenated sesame oil, coconut oil, flaxseed oil, cinnamon oil, clove oil, nutmeg oil, coriander oil, lemon oil, orange oil, safflower oil, cocoa butter, palm oil, palm kernel oil, sunflower oil, rapeseed oil, castor oil, hydrogenated castor oil, polyoxyethylene castor oil derivatives, borage oil, beeswax, lanolin, petroleum jelly, mineral oil and light mineral oil.

### The Poloxamer

The formulation according to the present invention comprises at least one poloxamer in an amount of 30 to 60 wt%, wherein the poloxamer is poloxamer 124 or poloxamer 188, or a mixture thereof.

A poloxamer is defined according to formula (II) 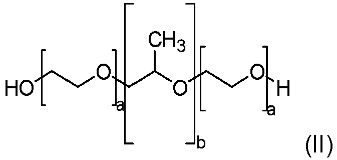 wherein a is an integer of from 10 to 110 and b is an integer of from 20 to 60.

When a is 12 and b is 20, this is known as poloxamer 124.

When a is 80 and b is 27, this is known as poloxamer 188.

The formulation may comprise two poloxamers. When the formulation comprises two poloxamers, it is preferred that they are poloxamer 124 and poloxamer 188.

Other known poloxamers useful in the present invention are poloxamer 237 (a = 64; and b = 37), poloxamer 338 (a = 141; and b = 44) and poloxamer 407 (a = 101; and b = 56).

Further poloxamers that are known and can be useful in the present invention include poloxamer 108, poloxamer 182, poloxamer 183, poloxamer 212, poloxamer 217, poloxamer 238, poloxamer 288, poloxamer 331, poloxamer 338 and poloxamer 335.

Preferably the total amount of poloxamer present is from about 40 to about 50 wt%. The total amount of poloxamer present may be about 45 wt%.

When the formulation comprises poloxamer 124 and poloxamer 188, the amount of poloxamer 124 may be 5 wt% and the amount of poloxamer 188 may be 40 wt%, based on the total composition.

In some cases, the formulation may comprise only one poloxamer, wherein the poloxamer is poloxamer 188.

It has been found that, when poloxamer 407 is used, it is preferred that poloxamer 124 is present.

It has been found that the formulation of the invention has excellent rehydration properties. The formulation rehydrates rapidly and homogeneously. Upon rehydration the formulation has excellent release properties.

It has been found that the formulation of the invention has excellent stability. Without wishing to be bound by theory, it is believed that the presence of at least one poloxamer in the formulation affords excellent stability.

### Additional Agents

The formulation may additionally comprise a flavouring agent, such as peppermint.

The formulation may additionally comprise a sweetener, such as sucrose.

The formulation may further comprise an antioxidant, preferably in an amount of from about 0.001 to 5 wt%, more preferably about 0.001 to 2.5 wt%, based on the total composition.

The antioxidant may be selected from the group consisting of butylated hydroxytoluene, butylated hydroxyl anisole, alpha- tocopherol (Vitamin E), ascorbyl palmitate, ascorbic acid, sodium ascorbate, ethylenediamino tetraacetic acid, cysteine hydrochloride, citric acid, sodium citrate, sodium bisulfate, sodium metabisulfite, lecithin, propyl gallate, sodium sulfate, monothioglycerol and mixtures thereof.

A preferred group of antioxidants is alpha- tocopherol (Vitamin E), monothioglycerol, ascorbic acid, citric acid and mixtures thereof. A preferred antioxidant is alpha- tocopherol (Vitamin E).

### Forms

The formulation according to the invention may be in an oral dosage form selected from the group consisting of mucoadhesive gel, a tablet, a powder, a liquid gel capsule, solid capsule, an oral solution, granule, or extrudates.

### Preferred Formulations

It is preferred that the type IV oral formulation according to the invention is a solid at room temperature and pressure, i.e. preferably the formulation is a solid at 20 °C and 1 atm. Such formulations are typically fluid during manufacture, solid at room temperature and become fluid again at 37 °C. For the purposes of the invention, a gel is considered to be a solid.

The formulation may comprise about 20 to 30 wt% solvent and two poloxamers, wherein the total amount of poloxamer is about 30 to 60 wt%, based on the pharmaceutical formulation.

Preferably the formulation comprises about 20 to 30 wt% total cannabinoid, about 20 to 30 wt% solvent and two poloxamers, wherein the total amount of poloxamer is about 30 to 60 wt%, based on the pharmaceutical formulation.

Preferably the formulation comprises THC; CBD; at least two poloxamers, wherein the poloxamers are poloxamer 124 and poloxamer 188; and a solvent, wherein the solvent is triethyl citrate. More preferably the formulation comprises about 20 to 30 wt% total cannabinoid; about 20 to 30 wt% triethyl citrate; and two poloxamers, wherein the poloxamers are poloxamer 124 and poloxamer 188, wherein the total amount of poloxamer is about 30 to 60 wt%, based on the pharmaceutical formulation.

In a highly preferred formulation, the formulation comprises THC; CBD in an amount of about 20 to 30 wt% total cannabinoid; about 20 to 30 wt% triethyl citrate; an anti-oxidant, wherein the antioxidant is alpha-tocopherol; and two poloxamers, wherein the poloxamers are poloxamer 124 and poloxamer 188, wherein the total amount of poloxamer is about 40 to 50 wt%, based on the pharmaceutical formulation. In this preferred formulation, the formulation is in the form of an oral dosage form, wherein the oral dosage form is a capsule.

### Treatment

The formulation is for use in therapy, preferably for use in paediatric epilepsy.

The formulation may also be used in the treatment of a disease or disorder selected from the group consisting of Dravet Syndrome, Lennox Gastaut Syndrome, myocolonic seizures, juvenile myocolonic epilepsy, refractory epilepsy, schizophrenia, juvenile spasms, West syndrome, infantile spasms,

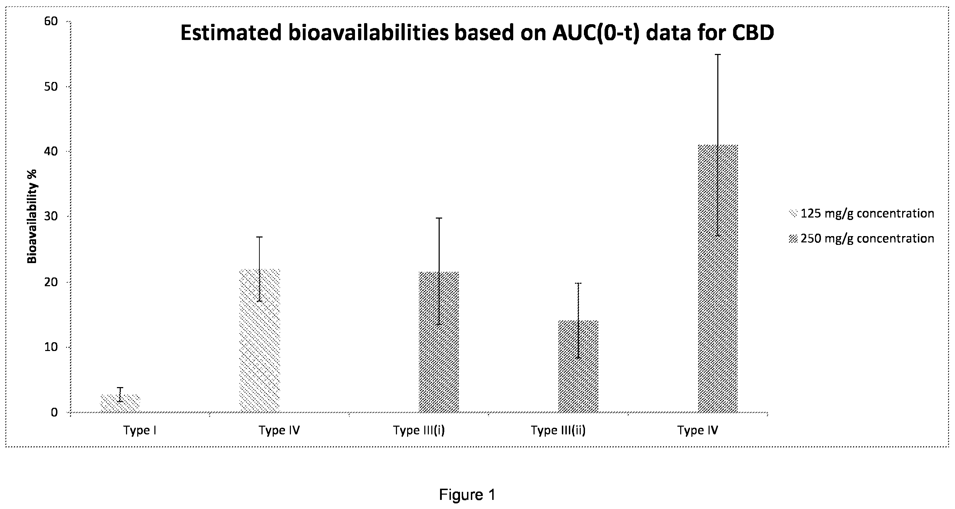

## Claims

1. An oral pharmaceutical formulation comprising tetrahydrocannabinol (THC); cannabidiol (CBD); a solvent in an amount of 20 to 50 wt%, wherein the solvent is selected from the group consisting of diacetin, propylene glycol, triacetin, monoacetin, propylene glycol diacetate, triethyl citrate and mixtures thereof; and at least one poloxamer in an amount of 30 to 60 wt%, wherein the poloxamer is poloxamer 124 or poloxamer 188, or a mixture thereof; and wherein the formulation is oil-free.

2. The formulation according to any one of the preceding claims, wherein the THC and CBD are synthetic or highly purified from their natural source.

3. The formulation according to any one of the preceding claims, wherein the ratio by weight of THC to CBD is in the range of from 100:1 to 1:100.

4. The formulation according to any one of the preceding claims, wherein the ratio by weight of the THC to CBD is 1:1.

5. The formulation according to any one of the preceding claims, wherein the total amount of cannabinoids is in an amount of from about 10 to 50 wt%.

6. The formulation according to any one of the preceding claims, wherein the formulation comprises two poloxamers.

7. The formulation according to claim 6, wherein the two poloxamers are poloxamer 124 and poloxamer 188.

8. The formulation according to any one of the preceding claims, wherein the solvent is selected from the group consisting of propylene glycol, propylene glycol diacetate, triethyl citrate and mixtures thereof.

9. The formulation according to any one of the preceding claims, wherein the solvent is selected from the group consisting of propylene glycol, triethyl citrate and mixtures thereof.

10. The formulation according to any one of the preceding claims, wherein the solvent is triethyl citrate.

11. The formulation according to any one of the preceding claims, wherein the solvent is present in an amount of from 20 to 30 wt%, based on the total composition,.

12. The formulation according to any one of the preceding claims, further comprising an antioxidant, in an amount of from 0.001 to 5 wt% based on the total composition.

13. The formulation according to claim 12, wherein the antioxidant is selected from the group consisting of butylated hydroxytoluene, butylated hydroxyl anisole, alpha-tocopherol (Vitamin E), ascorbyl palmitate, ascorbic acid, sodium ascorbate, ethylenediamino tetraacetic acid, cysteine hydrochloride, citric acid, sodium citrate, sodium bisulfate, sodium metabisulfite, lecithin, propyl gallate, sodium sulfate, monothioglycerol and mixtures thereof.

14. The formulation according to claim 13, wherein the antioxidant is selected from the group consisting of alpha- tocopherol (Vitamin E), monothioglycerol, ascorbic acid, citric acid and mixtures thereof.

15. The formulation according to any one of the preceding claims, wherein the formulation is a solid at 20 °C and 1 atm.

16. The formulation according to any one of the preceding claims, wherein the formulation is an oral dosage form selected from the group consisting of mucoadhesive gel, a tablet, a powder, a liquid gel capsule, solid capsule, an oral solution, granule, or extrudates.

## Patentansprüche

1. Orale pharmazeutische Formulierung, enthaltend Tetrahydrocannabinol (THC); Cannabidiol (CBD); ein Lösungsmittel in einer Menge von 20 bis 50 Gew.-%, wobei das Lösungsmittel ausgewählt ist aus der Gruppe bestehend aus Diacetin, Propylenglycol, Triacetin, Monoacetin, Propylenglycoldiacetat, Triethylcitrat und Gemischen davon; und mindestens ein Poloxamer in einer Menge von 30 bis 60 Gew.-%, wobei das Poloxamer Poloxamer 124 oder Poloxamer 188 oder ein Gemisch davon ist; und wobei die Formulierung ölfrei ist.

2. Formulierung nach einem der vorhergehenden Ansprüche, wobei das THC und CBD synthetisch oder hochgereinigt aus ihrer natürlichen Quelle sind.

3. Formulierung nach einem der vorhergehenden Ansprüche, wobei das Verhältnis nach Gewicht von THC zu CBD in dem Bereich von 100:1 bis 1:100 liegt.

4. Formulierung nach einem der vorhergehenden Ansprüche, wobei das Verhältnis nach Gewicht des THC zu CBD 1:1 beträgt.

5. Formulierung nach einem der vorhergehenden Ansprüche, wobei die Gesamtmenge an Cannabinoiden in einer Menge von etwa 10 bis 50 Gew.-% vorliegt.

6. Formulierung nach einem der vorhergehenden Ansprüche, wobei die Formulierung zwei Poloxamere enthält.

7. Formulierung nach Anspruch 6, wobei die zwei Poloxamere Poloxamer 124 und Poloxamer 188 sind.

8. Formulierung nach einem der vorhergehenden Ansprüche, wobei das Lösungsmittel ausgewählt ist aus der Gruppe bestehend aus Propylenglycol, Propylenglycoldiacetat, Triethylcitrat und Gemischen davon.

9. Formulierung nach einem der vorhergehenden Ansprüche, wobei das Lösungsmittel ausgewählt ist aus der Gruppe bestehend aus Propylenglycol, Triethylcitrat und Gemischen davon.

10. Formulierung nach einem der vorhergehenden Ansprüche, wobei das Lösungsmittel Triethylcitrat ist.

11. Formulierung nach einem der vorhergehenden Ansprüche, wobei das Lösungsmittel in einer Menge von 20 bis 30 Gew.-%, basierend auf der Gesamtzusammensetzung, vorliegt.

12. Formulierung nach einem der vorhergehenden Ansprüche, ferner beinhaltend ein Antioxidans in einer Menge von 0,001 bis 5 Gew.-% basierend auf der Gesamtzusammensetzung.

13. Formulierung nach Anspruch 12, wobei das Antioxidans ausgewählt ist aus der Gruppe bestehend aus butyliertem Hydroxyltoluol, butyliertem Hydroxylanisol, Alpha-Tocopherol (Vitamin E), Ascorbylpalmitat, Ascorbinsäure, Natriumascorbat, Ethylendiamintetraessigsäure, Cysteinhydrochlorid, Citronensäure, Natriumcitrat, Natriumbisulfat, Natriummetabisulfit, Lecithin, Propylgallat, Natriumsulfat, Monothioglycerin und Gemischen davon.

14. Formulierung nach Anspruch 13, wobei das Antioxidans ausgewählt ist aus der Gruppe bestehend aus Alpha-Tocopherol (Vitamin E), Monothioglycerol, Ascorbinsäure, Citronensäure und Gemischen davon.

15. Formulierung nach einem der vorhergehenden Ansprüche, wobei die Formulierung bei 20 °C und 1 atm ein Feststoff ist.

16. Formulierung nach einem der vorhergehenden Ansprüche, wobei die Formulierung eine orale Darreichungsform ist, die ausgewählt ist aus der Gruppe bestehend aus mucoadhäsivem Gel, einer Tablette, einem Pulver, einer Flüssiggelkapsel, einer festen Kapsel, einer oralen Lösung, Granulat oder Extrudaten.

## Revendications

1. Formulation pharmaceutique orale comprenant du tétrahydrocannabinol (THC) ; du cannabidiol (CBD) ; un solvant en une quantité de 20 à 50 % en poids, dans laquelle le solvant est choisi dans le groupe constitué de la diacétine, du propylène glycol, de la triacétine, de la monoacétine, du diacétate de propylène glycol, du citrate de triéthyle et de mélanges de ceux-ci ; et au moins un poloxamère en une quantité de 30 à 60 % en poids, dans laquelle le poloxamère est le poloxamère 124 ou le poloxamère 188 ou un mélange de ceux-ci ; et dans laquelle la formulation est exempte d'huile.

2. Formulation selon l'une quelconque des revendications précédentes, dans laquelle le THC et le CBD sont synthétiques ou hautement purifiés à partir de leur source naturelle.

3. Formulation selon l'une quelconque des revendications précédentes, dans laquelle le rapport en poids du THC au CBD est compris dans la plage allant de 100:1 à 1:100.

4. Formulation selon l'une quelconque des revendications précédentes, dans laquelle le rapport en poids du THC au CBD est de 1:1.

5. Formulation selon l'une quelconque des revendications précédentes, dans laquelle la quantité totale de cannabinoïdes est en une quantité d'environ 10 à 50 % en poids.

6. Formulation selon l'une quelconque des revendications précédentes, dans laquelle la formulation comprend deux poloxamères.

7. Formulation selon la revendication 6, dans laquelle les deux poloxamères sont le poloxamère 124 et le poloxamère 188.

8. Formulation selon l'une quelconque des revendications précédentes, dans laquelle le solvant est choisi dans le groupe constitué du propylène glycol, du diacétate de propylène glycol, du citrate de triéthyle et de mélanges de ceux-ci.

9. Formulation selon l'une quelconque des revendications précédentes, dans laquelle le solvant est choisi dans le groupe constitué du propylène glycol, du citrate de triéthyle et de mélanges de ceux-ci.

10. Formulation selon l'une quelconque des revendications précédentes, dans laquelle le solvant est le citrate de triéthyle.

11. Formulation selon l'une quelconque des revendications précédentes, dans laquelle le solvant est présent en une quantité de 20 à 30 % en poids, sur la base de la composition totale.

12. Formulation selon l'une quelconque des revendications précédentes, comprenant en outre un antioxydant, en une quantité de 0,001 à 5 % en poids, sur la base de la composition totale.

13. Formulation selon la revendication 12, dans laquelle l'antioxydant est choisi dans le groupe constitué de l'hydroxyltoluène butylé, de l'hydroxylanisole butylé, de l'alpha-tocophérol (vitamine E), du palmitate d'ascorbyle, de l'acide ascorbique, de l'ascorbate de sodium, de l'acide éthylènediaminotétraacétique, du chlorhydrate de cystéine, de l'acide citrique, du citrate de sodium, du bisulfate de sodium, du métabisulfite de sodium, de la lécithine, du gallate de propyle, du sulfate de sodium, du monothioglycérol et de mélanges de ceux-ci.

14. Formulation selon la revendication 13, dans laquelle l'antioxydant est choisi dans le groupe constitué de l'alpha-tocophérol (vitamine E), du monothioglycérol, de l'acide ascorbique, de l'acide citrique et de mélanges de ceux-ci.

15. Formulation selon l'une quelconque des revendications précédentes, dans laquelle la formulation est un solide à 20 °C et 1 atm.

16. Formulation selon l'une quelconque des revendications précédentes, dans laquelle la formulation est une forme posologique orale choisie dans le groupe constitué d'un gel mucoadhésif, d'un comprimé, d'une poudre, d'une capsule de gel liquide, d'une capsule solide, d'une solution orale, d'un granulé ou d'extrudats.
